(19) Europäisches Patentamt

European Patent Office

Office européen des brevets

(11) **EP 3 335 777 B1**

(12) **FASCICULE DE BREVET EUROPEEN**

(45) Date de publication et mention de la délivrance du brevet:
**07.08.2019 Bulletin 2019/32**

(21) Numéro de dépôt: **17205278.9**

(22) Date de dépôt: **04.12.2017**

(51) Int Cl.:
*B01D 17/00* (2006.01)   *C10G 33/06* (2006.01)
*C09K 8/584* (2006.01)   *B01D 17/12* (2006.01)
*B01D 69/02* (2006.01)   *B01D 71/02* (2006.01)
*B01D 71/06* (2006.01)   *C02F 1/44* (2006.01)
*C10G 33/00* (2006.01)   *E21B 43/16* (2006.01)
*G01N 11/02* (2006.01)   *G01N 33/24* (2006.01)
*C02F 101/32* (2006.01)   *C02F 103/36* (2006.01)
*G01N 11/00* (2006.01)   *G01N 13/02* (2006.01)

(54) **PROCEDE POUR LE TRAITEMENT D'UN LIQUIDE AQUEUX AU MOYEN D'UN FILTRE DETERMINE EN FONCTION DE LA TENSION INTERFACIALE DU LIQUIDE**

VERFAHREN ZUR BEHANDLUNG EINER WÄSSRIGEN FLÜSSIGKEIT MIT EINEM FILTER, DER ABGHÄNGIG VON DER GRENZFLÄCHENSPANNUNG DER FLÜSSIGKEIT BESTIMMT WIRD

METHOD FOR TREATING AN AQUEOUS LIQUID BY MEANS OF A FILTER DETERMINED ACCORDING THE INTERFACIAL TENSION OF THE LIQUID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.12.2016 FR 1662413**

(43) Date de publication de la demande:
**20.06.2018 Bulletin 2018/25**

(73) Titulaire: **IFP Energies nouvelles**
**92852 Rueil-Malmaison Cedex (FR)**

(72) Inventeurs:
• **ARGILLIER, Jean-Francois**
  **92500 RUEIL-MALMAISON (FR)**
• **BENOIT, Antoine**
  **78580 MAULE (FR)**
• **HENAUT, Isabelle**
  **92500 RUEIL-MALMAISON (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**US-A- 4 655 927        US-A1- 2009 241 496**
**US-A1- 2013 149 745    US-A1- 2014 332 462**

**Description**

La présente invention concerne le domaine du traitement de l'eau, notamment par filtration, en particulier dans le domaine de l'exploitation de gisement d'hydrocarbures.

Jusqu'à peu, l'exploitation d'un champ pétrolifère dit conventionnel se déroulait couramment en deux étapes : une première étape de récupération primaire uniquement basée sur la surpression présente au sein du réservoir, suivie d'une seconde étape utilisant généralement le procédé de « waterflooding » (balayage à l'eau). Ce procédé consiste à injecter de l'eau dans la formation souterraine afin de compenser la chute de pression au sein du réservoir, et donc remobiliser l'huile en place. Cette eau, ainsi que l'eau qui peut être initialement contenue dans la formation souterraine se retrouve dans les effluents pétroliers. Il est donc nécessaire de traiter ces effluents pétroliers de manière à récupérer uniquement les hydrocarbures. La première étape du traitement des effluents pétroliers consiste généralement à séparer l'eau et l'huile de façon gravitaire (par exemple au moyen d'un procédé de « Free-Water Knock-Out »). L'huile ainsi récupérée est dirigée vers des procédés de désalinisation et de déshydratation. De plus, l'eau séparée de l'huile n'est pas complètement propre (le procédé de séparation gravitaire n'est pas parfait) : elle contient notamment des gouttes d'huile et des impuretés. Pour retirer ces impuretés et les gouttes d'huile, l'eau est dirigée vers des procédés de traitement de l'eau, notamment des procédés de déshuilage. A l'issue des procédés de traitement de l'eau, la qualité de l'eau doit être suffisante pour répondre aux normes légales ou adaptée pour la réinjection dans la formation souterraine.

Actuellement, les pétroliers cherchent à optimiser la récupération des hydrocarbures. Cela peut être effectué en diminuant la saturation résiduelle en huile obtenue à l'issue du procédé de waterflooding, qui est, en moyenne, de 65 % pour les réservoirs préférentiellement mouillables à l'eau. Pour répondre à cet objectif, de nouveaux procédés, appelés récupération tertiaire par voie chimique (ou Chemical Enhanced Oil Recovery, cEOR pour récupération assistée des hydrocarbures par voie chimique), sont développés. Ces procédés sont basés sur l'ajout d'additifs dans l'eau injectée pour le balayage tels que des polymères, des tensioactifs, des alcalins ou une combinaison de ces additifs. Or, après percolation de cette solution jusqu'au puits producteur, il a été démontré que les propriétés de l'effluent produit en tête de puits sont modifiées par les additifs (polymères, tensioactifs et/ou alcalins), rendant les procédés de séparation actuels inefficaces.

En particulier, pour le traitement de l'eau, les opérations de filtration de l'eau sont réalisées habituellement avec des filtres ayant des pores dont les rayons sont choisis légèrement inférieurs aux rayons des gouttes d'huile contenues dans l'eau, de manière à retenir ces gouttes d'huile. Ce critère n'est cependant plus valide lorsque l'eau à nettoyer contient des principes tensioactifs et/ou des bases issus de la récupération assistée des hydrocarbures. En effet, dans ce cas, on constate que les gouttes d'huile peuvent traverser des pores de plus petit rayon que le diamètre des gouttes.

A titre d'exemple, une eau, contenant 200 ppm de gouttes de brut (c'est-à-dire d'huile) de diamètre 15 $\mu$m avec différentes concentrations de tensioactif (appelé également surfactant) C12Tab, a été filtrée sur un filtre de diamètre de pore de 5 $\mu$m. Il s'agit d'une filtration frontale pour cet exemple). Les résultats (teneur en huile du filtrat, c'est-à-dire la teneur en huile de l'eau filtrée) sont rassemblés dans le tableau 1, et montrent que la présence de surfactant rend la filtration inefficace au-delà de 200 ppm de surfactant.

Tableau 1 - Filtration sur une membrane de 5 $\mu$m

| Concentration C12TAB (ppm) | 50 | 100 | 200 | 500 |
|---|---|---|---|---|
| Teneur en huile du filtrat (ppm) | 21 | 25 | 167 | 197 |

Pour pallier ces inconvénients, la présente invention concerne un procédé de traitement d'un liquide aqueux comprenant des gouttes d'huile et un tensioactif et/ou une base. Pour ce procédé, on détermine un seuil de taille de pores d'un filtre en prenant en compte la tension interfaciale entre les gouttes d'huile et l'eau, et on choisit un filtre dont la taille des pores est inférieure ou égale à ce seuil pour la filtration du liquide aqueux. La prise en compte de la tension interfaciale, qui est liée à la présence de tensioactif et/ou de base, permet de prendre en compte la déformation des gouttes, et donc d'adapter les pores du filtre à la composition du liquide aqueux. Ainsi, il est possible de garder une filtration efficace du liquide aqueux.

Le document US 4 655 927 décrit un procédé pour le traitement d'un liquide aqueux contenant des gouttes d'huile et un tensioactif avec un additif.

**Le procédé selon l'invention**

L'invention concerne un procédé selon la revendication 1 le traitement d'un liquide aqueux, ledit liquide aqueux comportant des gouttes d'huile et au moins un tensioactif et/ou au moins une base. Pour ce procédé, on réalise les étapes suivantes :

a) on détermine la tension interfaciale entre lesdites gouttes d'huile et l'eau au sein dudit liquide aqueux, ladite tension interfaciale étant dépendante dudit tensioactif et/ou de ladite base ;

b) on détermine un seuil de taille des pores d'un filtre pour retenir lesdites gouttes d'huile dudit liquide aqueux, ledit seuil de taille des pores étant fonction de ladite tension interfaciale ; et

c) on traite ledit liquide aqueux par filtration au moyen d'un filtre dont la taille desdits pores est inférieure ou égale audit seuil de taille des pores.

On détermine ledit seuil de taille des pores dudit filtre au moyen d'un nombre capillaire seuil.

On détermine ledit seuil de taille desdits pores $R_{def}$ par une formule du type : $$R_{def} = Ca^* \frac{\Gamma}{\eta \dot{\gamma}},$$ avec $\eta$ la viscosité dudit liquide aqueux, $\dot{\gamma}$ ledit taux de cisaillement appliqué à une goutte d'huile, $\Gamma$ la tension interfaciale et $Ca^*$ ledit nombre capillaire seuil.

On détermine ledit nombre capillaire seuil en mettant en oeuvre les étapes suivantes :

i) on détermine la viscosité $\eta$ dudit liquide aqueux, le taux de cisaillement appliqué à une goutte d'huile $\dot{\gamma}$, le rayon de la goutte R et la tension interfaciale $\Gamma$ pour une filtration dudit liquide aqueux au sein d'un filtre dont les pores sont de taille déterminée ; et

ii) on calcule ledit nombre capillaire seuil $Ca^*$ au moyen d'une formule du type :

$$Ca^* \equiv \frac{\eta \dot{\gamma} R}{\Gamma}.$$

Avantageusement, on détermine ladite viscosité dudit liquide aqueux par mesure, notamment au moyen d'un rhéomètre.

De préférence, on détermine ladite tension interfaciale par mesure, ou en fonction de ladite concentration et du type dudit tensioactif et/ou de ladite base.

Selon une caractéristique, on détermine ledit taux de cisaillement en fonction du débit dudit liquide aqueux dans ledit filtre.

De manière avantageuse, ladite filtration est une filtration membranaire frontale ou tangentielle avec écoulement dudit liquide aqueux.

Conformément à un mode de réalisation, ledit filtre est une membrane polymère, ou métallique ou céramique.

De plus, l'invention concerne un procédé de traitement d'un effluent pétrolier. Pour ce procédé, on réalise les étapes suivantes :

a) on réalise une séparation des phases dudit effluent pétrolier, pour séparer au moins une phase liquide aqueuse, une phase liquide d'huile et une phase gaz ; et

b) on traite ladite phase liquide aqueuse au moyen du procédé de traitement d'un liquide aqueux selon l'une des caractéristiques précédentes, ledit liquide aqueux comprenant des gouttes d'huile et au moins un tensioactif et/ou une base dudit fluide injecté.

En outre, l'invention concerne un procédé de récupération assistée d'hydrocarbures au sein d'une formation souterraine. Pour ce procédé, on réalise les étapes suivantes :

a) on injecte un fluide dans ladite formation souterraine, ledit fluide injecté comprenant au moins un tensioactif et/ou une base ;

b) on récupère un effluent pétrolier de ladite formation souterraine, ledit effluent pétrolier comprenant au moins une partie dudit fluide injecté ;

c) on réalise une séparation des phases dudit effluent pétrolier, pour séparer au moins une phase liquide aqueuse, une phase liquide d'huile et une phase gaz ; et

d) on traite ladite phase aqueuse au moyen du procédé de traitement d'un liquide aqueux selon l'une des caractéristiques précédentes, ledit liquide aqueux comprenant des gouttes d'huile et au moins un tensioactif et/ou une base dudit fluide injecté.

**EP 3 335 777 B1**

**Description détaillée de l'invention**

La présente invention concerne un procédé de traitement d'un liquide aqueux. Le liquide aqueux, comprend essentiellement de l'eau, quelques gouttes d'huiles (par exemple jusqu'à 500 ppm), et au moins un tensioactif et/ou au moins une base (par exemple un alcalin). Le liquide peut comprendre d'autres éléments en petites quantités, tels que des polymères, des charges alourdissantes, etc.

Le procédé de traitement selon l'invention vise à limiter la quantité de gouttes d'huile dans l'eau. Cette limitation de la quantité d'huile dans l'eau est mise en oeuvre au moyen d'un filtre, le liquide circulant dans le filtre au travers de pores. Les pores permettent de limiter mécaniquement le passage des gouttes d'huile. Le liquide aqueux peut être un liquide issu d'une séparation gravitaire d'huile et d'eau d'un effluent pétrolier. Toutefois, le procédé selon l'invention est adapté à tout type de liquide aqueux comprenant des gouttes d'huile et au moins un tensioactif et/ou une base.

On rappelle qu'un tensioactif ou un surfactant est un composé qui modifie la tension superficielle entre deux surfaces. Les composés tensioactifs sont des molécules amphiphiles, c'est-à-dire qu'elles présentent deux parties de polarité différente, l'une lipophile (qui retient les matières grasses) et apolaire, l'autre hydrophile (miscible dans l'eau) et polaire. Ils permettent ainsi de stabiliser le mélange de deux phases non miscibles, en interagissant avec l'une apolaire (c'est-à-dire lipophile donc hydrophobe), par sa partie hydrophobe ; tandis qu'avec l'autre phase qui est polaire, il interagira par sa partie hydrophile. De manière non exhaustive, on peut citer les tensioactifs suivants : nonyl phénol ethoxylates (Triton X100), ester de sorbitan (Tween 20), alkyl bétaïnes (Mackamine C10), Sodium dodecyl benzene sulfonate (SDBS).

La base contenue dans l'eau peut être un alcalin. Selon un exemple non limitatif, la base peut être du $Na_2CO_3$, NaOH etc.

Selon l'invention, on définit une taille de seuil des pores (également appelé seuil de taille des pores, ou « pore throat » en anglais) pour permettre cette séparation des gouttes d'huile. Le terme taille définit une dimension du pore, en particulier un rayon (ou diamètre) du pore. Le seuil de taille de pores selon l'invention est défini en prenant en compte la tension interfaciale entre les gouttes d'huile et l'eau. On rappelle que la tension interfaciale est le rapport entre le travail réversible fourni pour étendre une interface de séparation fluide-fluide (ici huile et eau) et l'extension correspondante de l'interface, toutes choses étant égales par ailleurs. La tension interfaciale dépend notamment du type de l'huile, du type de tensioactif, la concentration du tensioactif, de la présence ou non de base, de la concentration en base. La prise en compte du tensioactif et/ou de la base permet de prendre en compte la déformabilité des gouttes d'huile, déformabilité qui permet aux gouttes d'huile de passer dans des pores du filtre de plus petit diamètre (cf. Tableau 1 dans l'introduction). Ainsi, le procédé selon l'invention permet de dimensionner un filtre efficace pour séparer les gouttes d'huile ; les pores sont dimensionnés en prenant en compte la déformabilité des gouttes.

Le procédé selon l'invention comporte les étapes suivantes :

1. Détermination de la tension interfaciale
2. Détermination du seuil de taille des pores du filtre
3. Filtration du liquide

1. Détermination de la tension interfaciale

Lors de cette étape, on détermine la tension interfaciale entre les gouttes d'huile et l'eau du liquide aqueux. Cette étape permet de prendre en compte le tensioactif et/ou la base présent(s) dans le liquide aqueux.

Selon une première mise en oeuvre de l'invention, on peut déterminer la tension interfaciale entre les gouttes d'huile et l'eau par mesure de la tension interfaciale, par exemple par des méthodes de mesure de gouttes pendantes ou gouttes tournantes suivant l'ordre de grandeur des tensions interfaciales.

Selon une deuxième mise en oeuvre de l'invention, on peut déterminer la tension interfaciale au moyen d'abaques ou de données fournies par le fabricant, et ce, en tenant compte du type de tensioactif, et de sa concentration dans le liquide aqueux.Avantageusement, la détermination de la tension interfaciale peut également prendre en compte la présence de base dans le liquide aqueux. En effet, la présence de base, par exemple la présence de $Na_2CO_3$, abaisse la tension interfaciale suivant la composition de l'huile (appelé aussi brut), en particulier pour les bruts réactifs, c'est-à-dire des bruts acides, lourds ou asphalténiques qui contiennent des acides saponifiables en présence de base.

2. Détermination du seuil de taille des pores

Lors de cette étape, on détermine le seuil de taille des pores d'un filtre, qui sera apte à retenir les gouttes d'huile du liquide aqueux en prenant en compte la déformabilité des gouttes, c'est-à-dire la tension interfaciale.

Classiquement, pour le seuil de taille des pores, on détermine un rayon ou un diamètre de pores. Ce seuil de taille des pores du filtre correspond au « rayon/diamètre déformable » des gouttes d'huile, en d'autres termes, le rayon

4

ou le diamètre équivalent d'une goutte d'huile soumise à la tension interfaciale déterminée. Généralement, le seuil de taille des pores est compris entre 0.001 et 20 μm.

Avantageusement, le seuil de taille des pores du filtre peut être dépendant du débit du liquide aqueux dans le filtre.

Selon un mode de réalisation de l'invention, on peut déterminer le seuil de taille des pores au moyen d'un nombre capillaire seuil. Avantageusement, le nombre capillaire seuil peut être obtenu par un test de filtration d'un liquide aqueux. Le test de filtration peut être réalisé au moyen d'un filtre dont on connaît le diamètre des pores, et de préférence avec un liquide aqueux comprenant pas ou peu de tensioactif et/ou de base.

Ensuite, on peut déterminer le seuil de taille des pores (par exemple le rayon $R_{def}$ ou le diamètre $D_{def}$) à partir du nombre capillaire seuil $Ca^*$ et de la tension interfaciale $\Gamma$ déterminée à l'étape précédente, par une formule du type :

$$R_{def} = Ca^* \frac{\Gamma}{\eta\dot{\gamma}}$$ ou, le cas échéant, $$D_{def} = 2Ca^* \frac{\Gamma}{\eta\dot{\gamma}},$$ avec $\eta$ la viscosité du liquide aqueux, $\dot{\gamma}$ le taux de cisaillement appliqué à une goutte d'huile (pouvant être dépendant du débit du liquide aqueux dans le filtre), $\Gamma$ la tension interfaciale déterminée à l'étape précédente et $Ca^*$ le nombre capillaire seuil.

Selon une mise en oeuvre de l'invention, on peut déterminer le nombre capillaire seuil au moyen d'un test, en mettant en oeuvre les étapes suivantes :

i) pour une filtration efficace (c'est-à-dire pour lequel la filtration des gouttes d'huile est assurée par le filtre) du liquide aqueux au sein d'un filtre dont les pores sont de taille déterminée on détermine :

- la viscosité $\eta$,
- le taux de cisaillement appliqué à une goutte d'huile $\dot{\gamma}$,
- le rayon de la goutte R (le cas échant son diamètre D) et
- la tension interfaciale $\Gamma$ déterminée à l'étape précédente ; et

ii) on calcule, pour ce test, le nombre capillaire seuil $Ca^*$ au moyen d'une formule du type : $$Ca^* = \frac{\eta\dot{\gamma}R}{\Gamma}$$ (ou le cas échéant $$Ca^* = \frac{\eta\dot{\gamma}D}{2\Gamma}$$ ).

La détermination de la viscosité peut être obtenue par mesure, notamment au moyen d'un rhéomètre. Selon un mode de réalisation de l'invention, la viscosité peut être considérée identique pour le cas test et pour le cas à filtrer. Dans ce cas, étant donné que ce paramètre apparaît à la fois dans l'équation de détermination du nombre capillaire seuil et dans l'équation du seuil de taille des pores, il n'est pas nécessaire de le connaître.

Le taux de cisaillement dépend notamment du débit volumique du liquide à travers le fluide. Par exemple le

$$\dot{\gamma} = \beta \frac{4Q}{S\varphi r_h}$$

taux de cisaillement peut être déterminé par une formule du type avec Q le débit volumique, $\varphi$ la porosité, S la section de filtration, $\beta$ un paramètre de calage expérimental et $r_h$ le rayon hydrodynamique des pores (cette formule est notamment illustrée plus en détail dans le document Chauveteau G.: "Rodlike Polymer Solution Flow through Fine Pores: Influence of Pore Size on the Rheological Behavior", J. Rheol., 26, 2, p. 111-142, 1982). Selon un mode de réalisation de l'invention, le taux de cisaillement peut être considéré identique pour le cas test et pour le cas à filtrer (car on considère le débit constant). Dans ce cas, étant donné que ce paramètre apparaît à la fois dans l'équation de détermination du nombre capillaire seuil et dans l'équation du seuil de taille des pores, il n'est pas nécessaire de le connaître.

Le rayon des gouttes d'huile (ou le cas échéant leur diamètre) pour l'exemple peut être déterminé à l'aide d'un granulomètre basé sur la diffusion de la lumière, ou tout moyen analogue.

3. Filtration du liquide aqueux

Lors de cette étape, on réalise une filtration du liquide aqueux au moyen d'un filtre dont la taille des pores est inférieure ou égale (de préférence strictement inférieure) au seuil de la taille des pores déterminé à l'étape précédente.

Afin d'augmenter l'efficacité de la filtration, la taille des pores du filtre peut être inférieure à 0.9 fois le seuil de taille des pores déterminé à l'étape précédente.

Selon une mise en oeuvre de l'invention, on réalise une filtration membranaire frontale ou tangentielle avec écoulement du liquide aqueux. La membrane de filtration peut être une membrane polymère, ou une membrane métallique, ou une membrane céramique.

Ainsi, à la fin de l'étape de filtration grâce au procédé selon l'invention, une grande partie des gouttes d'huile sont bloquées par le filtre, l'eau récupérée est de meilleure qualité, et nécessite peu de traitement supplémentaire pour être conforme aux normes environnementales en vigueur.

L'invention concerne également un procédé de traitement d'un effluent pétrolier. On appelle effluent pétrolier, un fluide récupéré par un puits de production dans un procédé de récupération des hydrocarbures d'une formation souterraine. Un effluent pétrolier, comporte généralement de l'huile (hydrocarbures sous forme liquide), du gaz (hydrocarbures sous forme gazeuse) et de l'eau, ainsi qu'au moins une partie d'un fluide balayage injecté dans la formation en vue de la récupération des hydrocarbures.

Le procédé de traitement d'un effluent pétrolier peut comprendre au moins les étapes suivantes :

a) on réalise une séparation des phases de l'effluent pétrolier, pour séparer au moins une phase liquide aqueuse, une phase liquide d'huile, et une phase gaz, cette séparation peut être une séparation gravitaire, par exemple du type « Free-Water Knock-Out ». En sortie de cette étape, le liquide aqueux comporte essentiellement de l'eau, des gouttes d'huile, et au moins un tensioactif et/ou une base.

b) on traite le liquide aqueux issu de la séparation au moyen d'un procédé de traitement de liquide aqueux selon l'une des caractéristiques décrites précédemment, en prenant en compte le tensioactif et/ou la base présent dans ce liquide aqueux. De cette manière, on rend l'eau de meilleure qualité.

Le procédé de traitement de l'effluent pétrolier peut comprendre en outre une étape de traitement du gaz issu de la séparation.

De plus, le procédé de traitement de l'effluent pétrolier peut comprendre des étapes de traitement de l'huile issu de la séparation. Ces étapes de traitement de l'huile peuvent être des étapes de déshydratation, de dessalage, etc.

En outre, l'invention concerne un procédé de récupération assistée des hydrocarbures d'une formation souterraine. Le procédé de récupération assistée des hydrocarbures comprend au moins les étapes suivantes :

a) on injecte un fluide dans la formation souterraine, par un puits injecteur, le fluide injecté comprenant au moins un tensioactif et/ou une base (par exemple un alcalin) ; le fluide injecté peut comprendre également des polymères;

b) on récupère un effluent pétrolier de la formation souterraine, par un puits producteur, l'effluent pétrolier comprenant au moins une partie du fluide injecté, c'est-à-dire une partie du tensioactif, des polymères et/ou des alcalins ;

c) on réalise une séparation des phases de l'effluent pétrolier, pour séparer au moins une phase liquide aqueuse, une phase liquide d'huile et une phase gaz ; cette séparation peut être une séparation gravitaire, par exemple du type « Free-Water Knock-Out ». En sortie de cette étape, le liquide aqueux comporte essentiellement de l'eau, des gouttes d'huile, et au moins un tensioactif et/ou une base ; et

d) on traite la phase aqueuse au moyen du procédé de traitement d'un liquide aqueux décrit précédemment, le liquide aqueux comprenant au moins un tensioactif et/ou une base du fluide injecté. De cette manière, on rend l'eau de meilleure qualité.

Le procédé de récupération assistée des hydrocarbures peut comprendre en outre une étape de traitement du gaz issu de la séparation.

De plus, le procédé de récupération assistée des hydrocarbures peut comprendre des étapes de traitement de l'huile issue de la séparation. Ces étapes de traitement de l'huile peuvent être des étapes de déshydratation, de dessalage, etc.

**Exemples de réalisation**

Les caractéristiques et avantages du procédé selon l'invention apparaîtront plus clairement à la lecture des exemples d'application ci-après.

*Premier exemple*

Le premier exemple se base sur l'exemple du Tableau 1 de l'introduction, avec une concentration de 200 ppm de tensioactif C12TAB. On rappelle que dans le Tableau 1, l'eau contenait 200 ppm de gouttes de brut (d'huile) de diamètre 15 $\mu$m (rayon 7.5 $\mu$m), les pores du filtre avaient un diamètre de 5 $\mu$m (rayon 2.5 $\mu$m). De plus, le filtre utilisé était efficace avec une concentration en tensioactif de 50 ppm (première colonne du tableau 1), c'est-à-dire avec une tension interfaciale de 3 mNm. La viscosité du liquide aqueux est d'environ 1 cP.

On applique le procédé selon l'invention :

1. la tension interfaciale des gouttes d'huile avec 200 ppm de tensioactif C12TAB est de 0.5 mNm.

2. On calcule le nombre capillaire seuil à partir de la première colonne du tableau 1, soit :

$$Ca^* = \frac{\eta\gamma\dot{D}}{\Gamma} = \frac{10^{-2}\dot{\gamma}15.10^{-6}}{3.10^{-3}} = \dot{\gamma}.5.10^{-5}$$

Le seuil de taille de pores (en diamètre) pour une concentration de tensioactif de 200ppm est alors de :

$$D_{def} = Ca^* \frac{\Gamma}{\eta\dot{\gamma}} = \dot{\gamma}.5.10^{-5}\frac{0.5^{-3}}{10^{-2}\dot{\gamma}} = 2,5.10^{-6}$$

Par conséquent, le diamètre seuil des pores est de 2.5 $\mu$m, soit un rayon seuil de 1.25 $\mu$m.

L'estimation de ce nouveau diamètre seuil permet de choisir des nouvelles membranes avec une taille de pore appropriée, c'est-à-dire des pores de diamètre inférieur à 2.5$\mu$m. Deux membranes de diamètres de pores égaux à 1.2 $\mu$m et 0.2 $\mu$m ont alors été utilisées et ont abouti à une qualité d'eau correcte (cf. Tableau 2), ce qui traduit une bonne efficacité du filtrage.

Tableau 2 - Premier exemple

| Diamètre des pores ($\mu$m) | 5 | 1.2 | 0.2 |
|---|---|---|---|
| Teneur en huile du filtrat (ppm) | 167 | 30 | 11 |

*Deuxième exemple*

A titre de deuxième exemple, le cas d'une eau contenant 200 ppm de gouttes de brut (d'huile) de diamètre 8 $\mu$m avec 7g/L de $Na_2CO_3$ a été filtrée. Les résultats sont rassemblés dans le Tableau 3 et montrent que la présence de base abaisse la tension interfaciale huile/eau et rend la filtration inefficace si la taille de pores n'est pas réduite au regard de la déformabilité conférée aux gouttes $D_{def}$ (0.3 $\mu$m pour la valeur moyenne) calculée par le procédé selon l'invention sur la base du nombre capillaire seuil du cas de l'eau sans alcalin (colonne de droite du tableau). On remarque que le filtre efficace dans ce cas est le filtre ayant des pores de diamètre 0.2 $\mu$m. Dans ce tableau, on indique des valeurs minimales, moyennes et maximales du diamètre de déformabilité des gouttes, de manière à prendre en compte une distribution de taille de gouttes, représentative des gouttes d'huile présentes dans le liquide aqueux.

Tableau 3 - Deuxième exemple

| Concentration $Na_2CO_3$ (g/L) | 7 | 7 | 7 | 0 |
|---|---|---|---|---|
| Tension interfaciale (mNm) | 1 | 1 | 1 | 27 |
| $D_{def}$ min/moyen/max ($\mu$m) | 0.04/0.3/1.1 | 0.04/0.3/1.1 | 0.04/0.3/1.1 | 1/8/30 |
| Diamètre des pores des filtres ($\mu$m) | 5 | 1.2 | 0.2 | 1.2 |
| Teneur en huile du filtrat (ppm) | 196 | 33 | 4 | 0.5 |

*Troisième exemple*

A titre de troisième exemple, le cas d'une eau contenant 200ppm de gouttes de brut (d'huile) de diamètre 8 $\mu$m avec 0.5g/L d'un surfactant alkyl éther sulfate de sodium et 7g/L de $Na_2CO_3$ (base) a été filtrée. Les résultats sont rassemblés dans le Tableau 4 et montrent que la présence de surfactant et de base abaisse la tension interfaciale brut/eau. La filtration devient inefficace si la taille de pores n'est pas réduite au regard de la déformabilité conférée aux gouttes $D_{def}$ calculée par le procédé selon l'invention sur la base du nombre capillaire seuil du cas de l'eau sans alcalin ni surfactant (colonne de droite du tableau 4). Pour cet exemple, un filtre avec des pores de diamètre inférieur à 0.06 $\mu$m permet une filtration efficace. Dans ce tableau, on indique des valeurs minimales, moyennes et maximales du diamètre de déformabilité des gouttes, de manière à prendre en compte une distribution de taille de gouttes, représentative des gouttes d'huile présentes dans le liquide aqueux.

Tableau 4 - Troisième exemple

| Concentration tensioactif (ppm) | 500 | 500 | 500 | 0 |
|---|---|---|---|---|
| Concentration $Na_2CO_3$ (g/L) | 7 | 7 | 7 | 0 |
| Tension interfaciale (mNm) | 0.2 | 0.2 | 0.2 | 27 |
| $D_{def}$ min/moyen/max ($\mu$m) | 0.007/0.06/0.22 | 0.007/0.06/0.22 | 0.007/0.06/0.22 | 8 |
| Diamètre des pores des filtres ($\mu$m) | 5 | 1.2 | 0.2 | 1.2 |
| Teneur en huile du filtrat (ppm) | 200 | 143 | 103 | 0.5 |

Ainsi, le procédé selon l'invention permet un traitement efficace de l'eau, grâce au choix d'un filtre adapté qui permet une élimination importante des gouttes d'huile contenues dans l'eau, quelque soient les autres composés présents dans l'eau.

## Revendications

1. Procédé pour le traitement d'un liquide aqueux, ledit liquide aqueux comportant des gouttes d'huile et au moins un tensioactif et/ou au moins une base, **caractérisé en ce qu'**on réalise les étapes suivantes :

    a) on détermine la tension interfaciale entre lesdites gouttes d'huile et l'eau au sein dudit liquide aqueux, ladite tension interfaciale étant dépendante dudit tensioactif et/ou de ladite base ;
    b) on détermine un seuil de taille des pores d'un filtre d'un rayon $R_{def}$ pour retenir lesdites gouttes d'huile dudit liquide aqueux, ledit seuil de taille des pores étant fonction de ladite tension interfaciale, par une formule du type : $R_{def} = Ca^* \frac{\Gamma}{\eta \dot{\gamma}},$ $R_{def}$ avec $\eta$ la viscosité dudit liquide aqueux, $\dot{\gamma}$ ledit taux de cisaillement appliqué à une goutte d'huile, $\Gamma$ la tension interfaciale et $Ca^*$ le nombre capillaire seuil. ; ledit nombre capillaire seuil étant déterminé en mettant en oeuvre les étapes suivantes :

    i) on détermine la viscosité $\eta$ dudit liquide aqueux, le taux de cisaillement appliqué à une goutte d'huile $\dot{\gamma}$, le rayon de la goutte R et la tension interfaciale $\Gamma$ pour une filtration dudit liquide aqueux au sein d'un filtre dont les pores sont de taille déterminée ; et
    ii) on calcule ledit nombre capillaire seuil $Ca^*$ au moyen d'une formule du type :

$$Ca^* = \frac{\eta \dot{\gamma} R}{\Gamma}.$$

    c) on traite ledit liquide aqueux par filtration au moyen d'un filtre dont la taille desdits pores est inférieure ou égale audit seuil de taille des pores.

2. Procédé selon la revendication 1, dans lequel on détermine ladite viscosité dudit liquide aqueux par mesure, notamment au moyen d'un rhéomètre.

3. Procédé selon l'une des revendications précédentes, dans lequel on détermine ladite tension interfaciale par mesure, ou en fonction de ladite concentration et du type dudit tensioactif et/ou de ladite base.

4. Procédé selon l'une des revendications précédentes, dans lequel on détermine ledit taux de cisaillement en fonction du débit dudit liquide aqueux dans ledit filtre.

5. Procédé selon l'une des revendications précédentes, dans lequel ladite filtration est une filtration membranaire frontale ou tangentielle avec écoulement dudit liquide aqueux.

6. Procédé selon la revendication 5, dans lequel ledit filtre est une membrane polymère, ou métallique ou céramique.

7. Procédé de traitement d'un effluent pétrolier, **caractérisé en ce qu'**on réalise les étapes suivantes :

a) on réalise une séparation des phases dudit effluent pétrolier, pour séparer au moins une phase liquide aqueuse, une phase liquide d'huile et une phase gaz ; et

b) on traite ladite phase liquide aqueuse au moyen du procédé de traitement d'un liquide aqueux selon l'une des revendications précédentes, ledit liquide aqueux comprenant des gouttes d'huile et au moins un tensioactif et/ou une base dudit fluide injecté.

8. Procédé de récupération assistée d'hydrocarbures au sein d'une formation souterraine, **caractérisé en ce qu'**on réalise les étapes suivantes :

a) on injecte un fluide dans ladite formation souterraine, ledit fluide injecté comprenant au moins un tensioactif et/ou une base ;

b) on récupère un effluent pétrolier de ladite formation souterraine, ledit effluent pétrolier comprenant au moins une partie dudit fluide injecté ;

c) on réalise une séparation des phases dudit effluent pétrolier, pour séparer au moins une phase liquide aqueuse, une phase liquide d'huile et une phase gaz ; et

d) on traite ladite phase aqueuse au moyen du procédé de traitement d'un liquide aqueux selon l'une des revendications 1 à 6, ledit liquide aqueux comprenant des gouttes d'huile et au moins un tensioactif et/ou une base dudit fluide injecté.

**Patentansprüche**

1. Verfahren zur Behandlung einer wässrigen Flüssigkeit, wobei die wässrige Flüssigkeit Öltropfen und mindestens ein Tensid und/oder mindestens eine Base umfasst, **dadurch gekennzeichnet, dass** die folgenden Schritte durchgeführt werden:

a) Bestimmung der Grenzflächenspannung zwischen den Öltropfen und dem Wasser innerhalb der wässrigen Flüssigkeit, wobei die Grenzflächenspannung von dem Tensid und/oder der Base abhängt;

b) Bestimmung eines Größengrenzwerts der Poren eines Filters mit einem Radius $R_{def}$, um die Öltropfen der wässrigen Flüssigkeit zurückzuhalten, wobei der Größengrenzwert der Poren von der Grenzflächenspannung abhängt, durch eine Formel des Typs:

$$R_{def} = Ca^* \frac{\Gamma}{\eta \dot{\gamma}}$$

wobei $\eta$ die Viskosität der wässrigen Flüssigkeit ist, Y die Scherrate ist, die an einem Öltropfen angewandt wird, $\Gamma$ die Grenzflächenspannung ist, und *Ca\** die Grenzkapillarzahl ist; wobei die Kapillarzahl durch Einsatz der folgenden Schritte bestimmt wird:

i) Bestimmung der Viskosität η der wässrigen Flüssigkeit, der an einem Öltropfen Y angewandten Scherrate, des Radius des Tropfens R und der Grenzflächenspannung Γ für eine Filterung der wässrigen Flüssigkeit in einem Filter, dessen Poren von bestimmter Größe sind; und

ii) Berechnung der Grenzkapillarzahl Ca* mit Hilfe einer Formel des Typs:

$$Ca^* = \frac{\eta \dot{\gamma} R}{\Gamma}$$

c) Behandlung der wässrigen Flüssigkeit durch Filterung mit Hilfe eines Filters, dessen Porengröße kleiner oder gleich dem Porengrößengrenzwert ist.

2. Verfahren nach Anspruch 1, bei dem die Viskosität der wässrigen Flüssigkeit durch Messen insbesondere mit Hilfe eines Rheometers bestimmt wird.

3. Verfahren nach einem der vorhergehenden Ansprüche, bei dem die Grenzflächenspannung durch Messen oder in Abhängigkeit von der Konzentration und dem Typ des Tensids und/oder der Base bestimmt wird.

**4.** Verfahren nach einem der vorhergehenden Ansprüche, bei dem die Scherrate in Abhängigkeit von der Menge der wässrigen Flüssigkeit im Filter bestimmt wird.

**5.** Verfahren nach einem der vorhergehenden Ansprüche, bei dem die Filterung eine Frontal- oder Tangentialmembranfilterung mit Abfließen der wässrigen Flüssigkeit ist.

**6.** Verfahren nach Anspruch 5, bei dem der Filter eine Polymer- oder Metall- oder Keramikmembran ist.

**7.** Verfahren zur Behandlung eines Erdölproduktes, **dadurch gekennzeichnet, dass** die folgenden Schritte durchgeführt werden:

a) Durchführen einer Trennung der Phasen des Erdölproduktes, um mindestens eine wässrige flüssige Phase, eine flüssige Ölphase und eine Gasphase zu trennen; und
b) Behandeln der wässrigen flüssigen Phase mit Hilfe des Verfahrens zur Behandlung einer wässrigen Flüssigkeit nach einem der vorhergehenden Ansprüche, wobei die wässrige Flüssigkeit Öltropfen und mindestens ein Tensid und/oder eine Base des injizierten Fluids umfasst.

**8.** Verfahren zur unterstützten Rückgewinnung von Kohlenwasserstoffen in einer unterirdischen Formation, **dadurch gekennzeichnet, dass** die folgenden Schritte durchgeführt werden:

a) Injizieren eines Fluids in die unterirdische Formation, wobei das injizierte Fluid mindestens ein Tensid und/oder eine Base umfasst;
b) Rückgewinnung eines Erdölproduktes der unterirdischen Formation, wobei das Erdölprodukt mindestens einen Teil des injizierten Fluids umfasst;
c) Durchführen einer Trennung der Phasen des Erdölproduktes, um mindestens eine wässrige flüssige Phase, eine flüssige Ölphase und eine Gasphase zu trennen; und
d) Behandeln der wässrigen Phase mit Hilfe des Verfahrens zur Behandlung einer wässrigen Flüssigkeit nach einem der Ansprüche 1 bis 6, wobei die wässrige Flüssigkeit Öltropfen und mindestens ein Tensid und/oder eine Base des injizierten Fluids umfasst.

**Claims**

**1.** A method for treating an aqueous liquid, said aqueous liquid comprising oil droplets and at least one surfactant and/or at least one base, wherein the following steps are carried out:

a) determining the interfacial tension between said oil droplets and the water in said aqueous liquid, said interfacial tension being dependent upon said surfactant and/or said base;
b) determining a pore size threshold of a filter of radius $R_{def}$ for retaining said oil droplets from said aqueous liquid, said pore size threshold being a function of said interfacial tension; with a formula of the type :

$$R_{def} = Ca^* \frac{\Gamma}{\eta \dot{\gamma}}$$

where $\eta$ is the viscosity of the aqueous liquid, $\gamma$ is the shear rate applied to an oil droplet, r is the interfacial tension and *Ca\*·* is the threshold capillary number, said threshold capillary number *Ca\*.* being determined by carrying out the following steps :

i) determining the viscosity $\eta$ of said aqueous liquid, the shear rate applied to an oil droplet $\gamma$, the droplet radius R and the interfacial tension $\Gamma$ for filtration of said aqueous liquid in a filter whose pores are of a specified size; and
ii) Calculating said threshold capillary number Ca\* using a formula of the type:

$$Ca^* = \frac{\eta \dot{\gamma} R}{\Gamma}$$

c) treating said aqueous liquid by filtration using a filter for which the size of said pores is less than or equal to said pore size threshold.

2. The method as claimed in claim 1 , wherein said viscosity of said aqueous liquid is determined by measurement, notably using a rheometer.

3. The method as claimed in one of the preceding claims, wherein said interfacial tension is determined by measurement, or as a function of said concentration and of the type of said surfactant and/or of said base.

4. The method as claimed in one of the preceding claims, wherein said shear rate is determined as a function of the flow rate of said aqueous liquid in said filter.

5. The method as claimed in one of the preceding claims, wherein said filtration is a dead-end or tangential membrane filtration with flow of said aqueous liquid.

6. The method as claimed in claim 5, wherein said filter is a polymeric, or metallic or ceramic membrane.

7. A method for treating a petroleum effluent, wherein the following steps are carried out:

a) separating the phases of said petroleum effluent, to separate at least an aqueous liquid phase, a liquid oil phase and a gas phase; and
b) treating said aqueous liquid phase by the method for treating an aqueous liquid according to one of the preceding claims, said aqueous liquid comprising oil droplets and at least a surfactant and/or base of said injected fluid.

8. A method for assisted recovery of hydrocarbons from an underground format ion, where in the following steps are carried out:

a) injecting a fluid into said underground formation, said injected fluid comprising at least one surfactant and/or base;
b) recovering a petroleum effluent from said underground formation , said petroleum effluent comprising at least one part of said injected fluid;
c) separating the phases of said petroleum effluent, to separate at least an aqueous liquid phase, aliquid oil phase and agas phase; and
d) treating said aqueous phase by the method for treating an aqueous liquid as claimed in one of claims 1 to 6, said aqueous liquid comprising oil droplets and at least one surfactant and/or base of said injected fluid.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4655927 A

**Littérature non-brevet citée dans la description**

- **CHAUVETEAU G.** Rodlike Polymer Solution Flow through Fine Pores: Influence of Pore Size on the Rheological Behavior. *J. Rheol.,* 1982, vol. 26 (2), 111-142